# EUROPÄISCHE PATENTANMELDUNG

(11) **EP 4 851 297 A1**
(43) Veröffentlichungstag der Anmeldung: **09.12.2026**
(21) Anmeldenummer: 25181514.8
(22) Anmeldetag: 06.06.2025
(51) Int. Cl.: C12P 7/26, C12N 9/04, C12P 7/42

(54) **VERFAHREN ZUR HERSTELLUNG VON 5-KETO-D-GLUCONAT**

(71) Anmelder: Annikki GmbH, 8074 Raaba-Grambach (AT)
(72) Erfinder: Staunig, Nicole, 8074 Raaba-Grambach (AT)
(74) Vertreter: Schwarz & Partner Patentanwälte GmbH

(57) **Zusammenfassung**

Verfahren zur Herstellung von 5-Keto-D-Gluconat durch Oxidation von D-Glucose, welche in einer wässrigen Lösung vorliegt, gekennzeichnet durch die Kombination der Maßnahmen,
dass die D-Glucose durch Behandeln mit einer NAD(P)⁺-abhängigen Glucose-Dehydrogenase und einer NAD(P)⁺-abhängigen Gluconat-5-Dehydrogenase zu 5-Keto-D-Gluconat oxidiert wird, wobei reduzierter Kofaktor NAD(P)H gebildet wird,
dass der reduzierte Kofaktor NAD(P)H enzymatisch mit einer NAD(P)H-Oxidase und/oder einer NAD(P)H-abhängigen Alkoholdehydrogenase zu NAD(P)⁺ oxidiert wird, wobei Sauerstoff und/oder eine Keto-Verbindung als Kosubstrat für die Oxidation des reduzierten Kofaktors eingesetzt wird, und
dass die Glucose-Dehydrogenase, die Gluconat-5-Dehydrogenase und die Keto-Verbindung in der wässrigen Lösung gelöst vorliegen. (Figur 1)

## Beschreibung

Die Erfindung betrifft ein Verfahren zur Herstellung von 5-Keto-D-Gluconat aus D-Glucose.

### Hintergrund der Erfindung

5-Keto-D-Gluconsäure/5-Keto-D-Gluconat (5KGA), ein Oxidationsprodukt der D-Gluconsäure (D-Gluconat), wird als Antioxidans und Säuerungsmittel in der Lebensmittelindustrie sowie als chiraler Building Block eingesetzt (Kataoka, 2024; Keliang & Dongzhi, 2006). Darüber hinaus kann 5KGA katalytisch (z.B. mit CuSO₄) in L-(+)-Weinsäure umgewandelt werden, welche Anwendungen in der Lebensmittel- und Textilindustrie sowie als Reagenz in der organischen Chemie hat (Sheng et al., 2024).

Überwiegend werden Essigsäurebakterien wie *Gluconobacter* sp. zur Herstellung von 5KGA aus D-Glucose in industriell etablierten fermentativen Verfahren eingesetzt. Diese zeichnen sich durch eine Vielzahl an membrangebundenen Dehydrogenasen, die in den periplasmatischen Raum ragen, aus.

D-Glucose wird zunächst von der membrangebundenen D-Glucose-Dehydrogenase (mGDH) zum D-Gluconat oxidiert, welches in weiterer Folge von der Glycerol-Dehydrogenase/D-Sorbitol-Dehydrogenase/D-Arabitol-Dehydrogenase (mGLDH/mSLDH/mARDH) zu 5-Keto-D-Gluconat oxidiert wird. Beide Enzyme sind abhängig vom Kofaktor PQQ (Pyrrolochinolinchinon) und als solche an die Atmungskette angebunden (Kataoka, 2024; Matsushita et al., 2003).

So verwendeten Sheng et al. (2024) einen *Gluconobacter oxydans*-Stamm in einem Fed-Batch-Verfahren ausgehend von D-Glucose, wo ein Titer von 179,4 g/I 5KGA und ein Umsatz von 92,5% nach ca. 94 h erreicht wurde.

Neben den membrangebunden Enzymen weist *Gluconobacter oxydans* ein weiteres System bestehend aus löslichen NAD(P)-abhängigen Enzymen im Zytoplasma auf - Glucose-Dehydrogenase (GDH) und Gluconat:NADP-5 Oxidoreduktase (GNO) (Keliang & Dongzhi, 2006; Herrmann et al., 2003).

Chen et al. (2023) nutzten ebenfalls ein lösliches Enzym (Gluconat-5-Dehydrogenase; Ga5DH) zur Oxidation von D-Gluconat zu 5-Keto-D-Gluconat. Die Autoren testeten zunächst die Ga5DH mit drei verschiedenen Regenerationssystemen für den Kofaktor NADP: eine Short Chain-Reduktase (PsCR) aus *Pichia stipitis,* eine NAD(P)H-Oxidase (LsNOX) sowie eine Cyclohexanon-Monooxygenase (CHMO). Ethyl-4-chlor-3-oxobutanoat (COBE), welches zu Ethyl-(*S*)-4-chlor-3-hydroxybutanoat ((S)-CHBE), einem Precursor der pharmazeutisch bedeutenden Statine, reduziert wird, wurde als Kosubstrat für die PsCR und Cyclohexanon als Kosubstrat für die CHMO eingesetzt. Unter den getesteten Bedingungen (100 mM D-Gluconat, 150 mM Kosubstrat (COBE oder Cyclohexanon), 0,2 mM NADP⁺ bei pH 8, 1 h) lieferte das System mit der PsCR die höchste katalytische Aktivität (ca. 43% 5KGA-Ausbeute) gefolgt von LsNOX (ca. 28% 5KGA-Ausbeute). Da laut den Autoren Ganzzell-Systeme leichter herzustellen, kosteneffizient, sehr tolerant gegenüber organischen Lösungsmitteln und daher sehr prozessstabil und darüber hinaus leicht abzutrennen und wiederzuverwenden sind, wurden zwei *E*. *coli*-Ganzzellkatalysatoren (Katalysator 1 exprimiert PsCR und Ga5DH; Katalysator 2 exprimiert PsCR und eine Glucose-Dehydrogenase) entwickelt. Schlussendlich konstruierten die Autoren mit den zwei Ganzzellkatalysatoren ein "Hantel-Oxidations"-System in einem biphasischen Reaktionsgemisch (Wasser und n-Butylacetat), bei dem zunächst das eingesetzte D-Gluconat (50 mM) in der wässrigen Phase mittels Katalysator 1 zu 5KGA oxidiert und COBE (100 mM) in der organischen Phase mittels Katalysator 1 zum (S)-CHBE reduziert wurde. 5KGA wurde als Calcium-Salz ausgefällt und die organische Phase wurde mit neuer wässriger Phase, die D-Glucose (100 mM) und Katalysator 2 enthielt, kombiniert. In einem weiteren Zyklus wurden 100 mM COBE und 50 mM D-Glucose eingebracht.

Nachteilig an diesem Verfahren ist, dass ein vollständiger Umsatz aller eingebrachten Substrate unmöglich erreicht werden kann, da nach jeder biokatalytischen Umsetzung und Abtrennung der gewünschten Produkte (5-Keto-D-Gluconat und (S)-CHBE) entweder eine Mischung aus COBE und (S)-CHBE (in der organischen Phase) oder D-Glucose und D-Gluconat (in der wässrigen Phase) übrig bleibt, die nur durch Zugabe von weiterem Substrat (in der entsprechenden komplementären Phase) umgesetzt werden können. Zusätzlich müssen die Ganzzellkatalysatoren nach jeder Umsetzung abgetrennt und für einen weiteren Durchgang wieder neu zugesetzt werden. Ein weiterer Nachteil ist die Verwendung von organischem Lösungsmittel (n-Butylacetat) als zweite Phase zur Lösung des Kosubstrats.

Hier setzt nun die folgende Erfindung an und möchte ein einfacheres Verfahren zur Herstellung von 5-Keto-D-Gluconat zur Verfügung stellen.

### Detaillierte Beschreibung der Erfindung

Die Aufgabe zur Herstellung von 5-Keto-D-Gluconat aus D-Glucose wird in einem Verfahren gelöst, welches gekennzeichnet ist durch die Kombination der Maßnahmen, dass die D-Glucose durch Behandeln mit einer NAD(P)'-abhängigen Glucose-Dehydrogenase und einer NAD(P)'-abhängigen Gluconat-5-Dehydrogenase zu 5-Keto-D-Gluconat oxidiert wird, wobei reduzierter Kofaktor NAD(P)H gebildet wird, dass der reduzierte Kofaktor NAD(P)H enzymatisch mit einer NAD(P)H-Oxidase und/oder einer NAD(P)H-abhängigen Alkoholdehydrogenase zu NAD(P)⁺ oxidiert wird, wobei Sauerstoff und/oder eine Keto-Verbindung als Kosubstrat für die Oxidation des reduzierten Kofaktors eingesetzt wird, und dass die Glucose-Dehydrogenase, die Gluconat-5-Dehydrogenase und die Keto-Verbindung in der wässrigen Lösung gelöst vorliegen.

"Keto-Verbindung" bezeichnet in diesem Zusammenhang eine organische Verbindung mit einer Keto-Gruppe, also z. B. Aldehyde und Ketone.

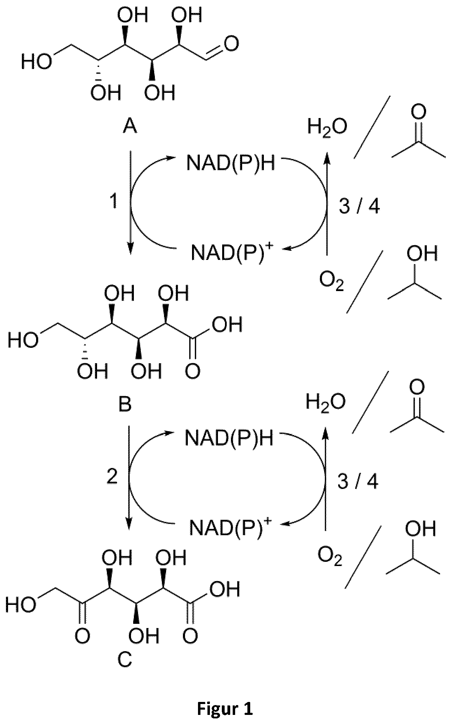

Die Figur 1 zeigt die eine bevorzugte Ausführungsform des erfindungsgemäßen Verfahrens. Die Bezeichnung A steht für D-Glucose, B für D-Gluconsäure/D-Gluconat, C für 5-Keto-D-gluconsäure/5-Keto-D-gluconat, 1 für Glucose-Dehydrogenase, 2 für Gluconat-5-Dehydrogenase, 3 für NAD(P)H-Oxidase und 4 für Alkoholdehydrogenase.

Es hat sich überraschenderweise gezeigt, dass durch den Einsatz einer NAD(P)H-Oxidase oder einer NAD(P)H-abhängigen Alkoholdehydrogenase zur Oxidation von NAD(P)H die oben genannten Nachteile nicht auftreten. So kann die Umsetzung in einer einphasigen wässrigen Lösung durchgeführt werden und im Laufe der Reaktion muss kein weiteres Kosubstrat nachgegeben werden, um eine vollständige Umsetzung der D-Glucose zu 5-Keto-D-Gluconat zu erreichen.

In einer bevorzugten Ausführungsform wird das Verfahren zur Herstellung von 5-Keto-D-Gluconat als Eintopf-Verfahren durchgeführt, das bedeutet, dass alle Reaktionsschritte im selben Reaktionsgefäß (Reaktor) durchgeführt werden.

Bevorzugt werden eine einzige NAD(P)H-Oxidase und/oder eine einzige NAD(P)H-abhängigen Alkoholdehydrogenase zur Oxidation des reduzierten Kofaktors NAD(P)H eingesetzt.

Besonders bevorzugt wird eine einzige NAD(P)H-Oxidase zur Oxidation des reduzierten Kofaktors NAD(P)H eingesetzt.

In einer besonders bevorzugten Ausführungsform des erfindungsgemäßen Verfahrens ist die Keto-Verbindung Aceton.

Besonders bevorzugt wird das vorliegende Verfahren außerhalb eines Organismus durchgeführt (in *vitro*)*,* das heißt nicht in einer ganzen Zelle und auch nicht fermentativ.

Besonders bevorzugt wird das Verfahren in einer einzigen flüssigen Phase durchgeführt.

In weiteren bevorzugten Ausführungsform des erfindungsgemäßen Verfahrens beträgt die Konzentration der Substrate in der wässerigen Lösung zwischen 5 und 250 g/l.

Der besonders bevorzugte Temperaturbereich der Reaktionen liegt zwischen 15 und 50 °C.

Der besonders bevorzugte pH-Bereich der Reaktionen liegt zwischen pH 6 und 10,5.

Bei einer weiteren, bevorzugten Variante des erfindungsgemäßen Verfahrens liegen die Enzyme in einer Suspension, im Homogenat und/oder im Lysat der entsprechenden, sie bildenden Zellen vor, wobei Lysate besonders bevorzugt sind. Zudem können die Enzyme auch in lyophilisierter oder sprühgetrockneter oder immobilisierter Form im erfindungsgemäßen Verfahren eingesetzt werden. Die erfindungsgemäß eingesetzten Enzyme können auch in einer im Wesentlichen gereinigten Form verwendet werden, so dass die Enzympräparate keine oder im Wesentlichen keine Bestandteile umfassen, die von den Zellen bzw. der Kultur, in der diese exprimiert wurden, stammen. Die beispielsweise durch rekombinante Expression (z.B. in *E. coli*) hergestellten Enzyme können durch Verfahren, die im Stand der Technik bekannt sind, gereinigt werden, um nach u.a. Ultrafiltration und DNA-Entfernung auch in und zur Herstellung von Lebensmitteln eingesetzt zu werden.

Suspension bedeutet in diesem Kontext eine Suspension von *resting cells.* Diese werden nach der Kultivierung geerntet (vom Nährmedium abgetrennt) und in einem geeigneten Puffersystem suspendiert. Im Gegensatz zu fermentativen Verfahren, bei denen auch mit ganzen Zellen gearbeitet wird, können die *resting cells* aufgrund der Entfernung von Kohlenstoff-Quellen und Nährstoffen nicht mehr wachsen, sondern dienen nur der Umsetzung von Substraten (Lin & Tao, 2017). Homogenat steht in diesem Kontext für eine physikalisch und/oder chemisch behandelte Suspension (z.B. mittels Druckes, Lysozym oder Ultraschall behandelt), wobei die Zellbestandteile aus den Zellen freigesetzt werden. Ein Lysat wird erhalten, wenn die unlöslichen Zellbestandteile des Homogenats beispielsweise durch Filtration oder Zentrifugation entfernt werden (siehe *Produktion der Enzyme & Herstellung der Lysate* für Details).

In einer weiteren Variante können die Enzyme auch mit einem wasserlöslichen Polymer wie Polyethylenglycol am N-Terminus modifiziert, in oder auf einer festen Matrix immobilisiert oder Teil eines Fusionsproteins sein.

Mit den nachfolgenden Beispielen werden bevorzugte Ausführungsformen der Erfindung noch näher beschrieben.

### Materialien

Triethanolamin und Natriumdodecylsulfat (SDS) wurden von Carl Roth, 2-Propanol wurde von Honeywell, IPTG (Isopropyl-β-D-thiogalactopyranosid), D-Glucose, Natrium-D-Gluconat und Kalium-5-Keto-D-Gluconat wurden von Sigma-Aldrich und NAD⁺, NADH-Dinatriumsalz, NADP⁺-Dinatriumsalz, NADPH-Tetranatriumsalz sowie Acetonitril wurde von PanReac AppliChem (ITW Reagents) bezogen.

### Produktion der Enzyme & Herstellung der Lysate

### Allgemeines zur Expression von rekombinanten Enzymen in E. coli

Für die rekombinante Enzymproduktion in einem *Escherichia coli*-Stamm wurde zunächst das zu exprimierende Gen in einer PCR unter der Verwendung der genomischen DNA oder deren synthetisch an die Codon-Verwendung von *E. coli* angepasstes Äquivalent als Matrize zusammen mit spezifischen Oligonukleotiden, die zusätzlich Erkennungssequenzen für Restriktionsendonukleasen tragen, amplifiziert und aus dem Reaktionsgemisch isoliert. Nach dem Nukleinsäure-Verdau mit den Restriktionsenzymen Sphl und HindIII wurde das für das Target-Enzym kodierende Genfragment in das mit Sphl und Hindlll geschnittene Rückgrat des Expressionsvektors pQE70-Kan ligiert. Das Ligationsprodukt wurde in chemisch kompetente *E*. *coli*-Zellen Top10F' transformiert und die entstandenen Kolonien wurden für die Plasmid-Isolierung und Restriktionsanalyse benutzt.

Das Ergebnis des Klonierungs-Schritts wurde mittels Restriktionsenzym-Verdau und DNA-Sequenzierung überprüft. Das resultierende Konstrukt trägt das Target-Gen unter dem IPTG-induzierbaren T5-Promotor.

Für die Überexpression des Enzymes in *E. coli* wurde das resultierende Expressionsplasmid in die kompetenten Expressionszellen RB791 transformiert. Nach 24 h Inkubation bei 37 °C wurden entstandene Kolonien für die Expressionstests in LB-Medium angeimpft.

Am nächsten Tag wurden damit Expressionskulturen mit einer optischen Dichte OD₅₅₀ von 0,02 angeimpft und bei 37 °C geschüttelt, bis eine OD₅₅₀ von 0,3 erreicht wurde. Anschließend wurde die Temperatur auf 25 °C gesenkt und die Kulturen wurden beim Erreichen einer OD₅₅₀ von 0,5 mit 0,1 mM IPTG induziert. Nach 22 h wurden die Kulturen geerntet (vom Medium mittels Zentrifugation in Form eines Zellpellets abgetrennt) und auf die Expression des rekombinanten Enzymes mit Hilfe von SDS-Gelelektrophorese und einer Aktivitätsbestimmung (Verwendung in Use-Test oder optisch-enzymatischer Assay) analysiert.

### Herstellung von Zell-Lysaten mittels Sonifier-Aufschlusses

Zur Herstellung einer Zell-Suspension wurde das nach obigen Verfahren hergestellte Zellpellet in einem geeigneten Gefäß eingewogen und mit Puffer und Lysozym (finale Konzentration 0,5 mg/ml) versetzt (z.B. Triethanolamin (TEA) - HCl) und unter Rührung gelöst. Der Massenanteil an Biomasse beträgt üblicherweise 20%, der Rest entfällt auf den Puffer.

Zum Zell-Aufschluss wurde ein Branson Sonifier 450 verwendet. Die Suspension wurde dreimal mit je 15 Ultraschall-Stößen (Einstellungen am Gerät: Timer = 15; Duty Cycle = 50; Output Control = 3 - 5) behandelt.

Das erhaltene Homogenat wurde 10 min lang bei 4 °C und 16000 rpm zentrifugiert (Eppendorf Zentrifuge 5417R), um die unlöslichen Zellfragmente abzutrennen und das Lysat zu erhalten.

**Tabelle 1. Enzymtypen und Spenderorganismen für die in den Beispielen verwendeten Enzyme.**

| **Enzymtyp (EC-Klasse)** | **katalysierte Reaktion** | **Spenderorganismus** | **Sequenzen/Literatur** |
|---|---|---|---|
| Glucose-Dehydrogenase I (GDH I) | D-Glucose → D-Gluconat | *Bacillus spizizenii* | (NCBI Protein Database: WP_003225027.1) |
| Glucose-Dehydrogenase II (GDH II) | | *Priestia megaterium* | (NCBI Protein Database: BAA01476.1) |
| Gluconat-5-Dehydrogenase I (Ga5DH I) | D-Gluconat → 5-Keto-D-Gluconat | *Gluconobacter oxydans* | (NCBI Protein Database: AHK72173.1) |
| Gluconat-5-Dehydrogenase II (Ga5DH II) | D-Gluconat → 5-Keto-D-Gluconat | *Pontivivens insulae* | (NCBI Protein Database: WP_108780707.1) |
| NAD(P)H-Oxidase | NAD(P)H → NAD(P)⁺ | *Carnobacterium divergens,* Mutante | WO 2025/012236 A1 |
| Alkoholdehydrogenase | Aceton → 2-Propanol | *Thermoanaerobacter brockii* | (NCBI Protein Database: CAA46053.1) |

### Analytische Methoden

### High Performance Liquid Chromatography (HPLC)

Zur Quantifizierung von D-Glucose mittels HPLC (High Performance Liquid Chromatography) wurde ein Agilent HPLC 1260 Infinity II Series System verwendet. Die Detektion erfolgte mittels eines Brechungsindex-Detektors (RI-Detektion). Zur Messung wurde eine Phenomenex Rezex RCM-Monosaccharide Ca+2 (8%) Säule mit entsprechender Vorsäule verwendet und mit 3,5% (v/v) 2-Propanol in Reinstwasser isokratisch eluiert.

### High Performance Anion Exchange Chromatography (HPAEC)

Zur Quantifizierung von D-Gluconat und 5-Keto-D-Gluconat mittels HPAEC (High Performance Anion Exchange Chromatography) wurde ein Dionex ICS6000 System mit AS-AP Autosampler verwendet. Die Messung erfolgte mittels Leitfähigkeitsdetektion (CD) gekoppelt an einen Dionex AERS 500 elektrolytisch regenerierten Suppressor im externen Wassermodus. Zur Trennung der Analyten wurde eine Dionex IonPac AS11-HC-4µm Säule mit entsprechender Vorsäule sowie einem NaOH-Gradienten verwendet. Das Laufmittel wurde zusätzlich mit einer Dionex ATC (Anion Trap Column) vorbehandelt.

### Bestimmung von Enzym-Aktivitäten (optisch-enzymatischer Assay)

Enzym-Aktivitäten in den Lysaten wurden mit einem Shimadzu UV-1900 Spektrophotometer bestimmt. Dazu wurde die Bildung oder der Verbrauch von NAD(P)H bei einer Wellenlänge von 340 nm über die Änderung der Absorption verfolgt. Die Messungen wurden mit 0,2 mM Kofaktor (NAD(P)⁺ oder NAD(P)H) durchgeführt. Dazu wurden 20 µl einer 10 mM Stock-Lösung des Kofaktors in einer Küvette (Greiner bioone Semi-Micro-Küvette aus Polystyrol) vorgelegt und der gewünschte pH-Wert wurde mit 100 mM TEA-HCl-Puffer eingestellt (870 µl). 10 µl Lysat (verdünnt oder unverdünnt) und 100 µl Substrat-Lösung wurden zur Küvette zugesetzt und die Messung unmittelbar danach gestartet. Die Messungen wurden standardmäßig bei 25 °C durchgeführt. Über den Extinktionskoeffizienten von NADH/NADPH bei 340 nm (*ε* = 6220 L mol⁻¹ cm⁻¹) kann die Enzym-Aktivität des Lysats in U/ml (bezogen auf das Volumen des Lysats) oder U/g (bezogen auf die zur Herstellung eingesetzte Biomasse) bestimmt werden. 1 U steht dabei für 1 µmol Substratumsatz pro Minute (1 U = 1 µmol/min = 1,67·10⁻⁸ kat).

Mit den nachfolgenden Beispielen werden bevorzugte Varianten des erfindungsgemäßen Verfahrens noch näher beschrieben. Die in diesen Beispielen eingesetzten Lysate wurden nach den oben beschriebenen Verfahren hergestellt.

### Beispiel 1

### Oxidation von D-Glucose zu 5-Keto-D-Gluconat

In acht 2 ml Glas-Vials wurden folgende Komponenten vermischt: 200 µl eines 500 mM TEA-HCl-Puffers (pH 8,5), 100 µl einer 278 mM D-Glucose-Lösung, 10 µl GDH-Lysat (siehe nachfolgende Tabelle 2), 10 µl Ga5DH-Lysat (siehe nachfolgende Tabelle 2), 5 µl einer 5 mM NADP⁺-Lösung (finale Konzentration 0,05 mM) sowie 10 U NAD(P)H-Oxidase-Lysat oder 10 U Alkoholdehydrogenase-Lysat. Alle Ansätze wurden mit deionisiertem Wasser auf 500 µl Gesamtvolumen aufgefüllt. Zusätzlich enthielten jene Ansätze mit Alkoholdehydrogenase 15 µl Aceton. Sämtliche Reaktionsteilnehmer liegen somit in einer einzigen flüssigen, im vorliegenden Fall wässrigen, Phase vor.

Die Ansätze wurden 20 h bei 30 °C unter kontinuierlichem Schütteln unter atmosphärischer Luft (1200 rpm, Eppendorf Thermomixer) inkubiert.

Zur Aufarbeitung wurden 100 µl eines Ansatzes mit 200 µl Acetonitril versetzt und bei 60 °C für 20 min inkubiert (1200 rpm, Eppendorf Thermomixer). Nach der Denaturierung wurde die Probe mit 700 µl Reinstwasser verdünnt und im Anschluss 10 min bei max. g zentrifugiert. 150 µl des klaren Überstands wurden in ein HPLC-Vial überführt und vermessen (HPLC, RI-Detektion). Für die Quantifizierung der Zuckersäuren wurde der Überstand 1:40 verdünnt und auf der HPAEC (Leitfähigkeitsdetektion) gemessen.

Die Ergebnisse sind der nachfolgenden Tabelle 2 dargestellt.

**Tabelle 2**

| **Kofaktor-Regeneration** | **Glucose-Dehydrogenase** | **Gluconat-5-Dehydrogenase** | **Ausbeute 5-Keto-D-Gluconat [%]** |
|---|---|---|---|
| NAD(P)H-Oxidase | GDH I | Ga5DH I | 92 |
| | | Ga5DH II | 88 |
| | GDH II | Ga5DH I | 91 |
| | | Ga5DH II | 87 |
| Alkoholdehydrogenase / Aceton | GDH I | Ga5DH I | 15 |
| | | Ga5DH II | 15 |
| | GDH II | Ga5DH I | 15 |
| | | Ga5DH II | 15 |

### Literatur

Kataoka, N. (2024). Ketogluconate production by Gluconobacter strains: enzymes and biotechnological applications. Bioscience, Biotechnology, and Biochemistry, 88(5), 499-508. https://doi.org/10.1093/bbb/zbae013
Keliang, G., & Dongzhi, W. (2006). Asymmetric oxidation by Gluconobacter oxydans. Applied Microbiology and Biotechnology, 70, 135-139. https://doi.org/10.1007/s00253-005-0307-0
Sheng, Z., Li, Y., Wang, J. (2024). High-yield production of 5-keto-D-gluconic acid via regulated fermentation strategy of Gluconobacter oxydans and its conversion to L-(+)-tartaric acid. Heliyon, 10(17), e36527. https://doi.org/10.1016/j.heliyon.2024.e36527
Matsushita, K., Fujii, Y., Ano, Y., Toyama, H., Shinjoh, M., Tomiyama, N., Miyazaki, T., Sugisawa, T., Hoshino, T., & Adachi, O. (2003). 5-Keto-D-Gluconate Production Is Catalyzed by a Quinoprotein Glycerol Dehydrogenase, Major Polyol Dehydrogenase, in Gluconobacter Species. Applied and Environmental Microbiology, 69(4), 1959-1966. https://doi.org/10.1128/AEM.69.4.1959-1966.2003
Herrmann, U., Merfort, M., Jeude, M., Bringer-Meyer, S., & Sahm, H. (2004). Biotransformation of glucose to 5-keto-D-gluconic acid by recombinant Gluconobacter oxydans DSM 2343. Applied Microbiology and Biotechnology, 64, 86-90. https://doi.org/10.1007/s00253-003-1455-8
Chen, J., Cai, J., Sha, F., Sun, W., Lyu, X., Chang, Y., Cao, F., Zhao, L., Wu, H., & Ouyang, P. (2023). Chemoenzymatic cascades producing 2,5-furandicarboxylic acid precursors via D-gluconate "barbell oxidation" and dehydration. Green Chemistry, 25(18), 7126-7140. https://doi.org/10.1039/D3GC01493B
Lin, B., & Tao, Y. (2017). Whole-cell biocatalysts by design. Microbial Cell Factories, 16, 106. https://doi.org/10.1186/s12934-017-0724-7
Protein [Internet]. Bethesda (MD): National Library of Medicine (US), National Center for Biotechnology Information. Accession No. WP_003225027.1, glucose 1-dehydrogenase [Bacillus spizizenii]. Verfügbar unter: https://www.ncbi.nlm.nih.gov/protein/WP_003225027.1
Protein [Internet]. Bethesda (MD): National Library of Medicine (US), National Center for Biotechnology Information. Accession No. BAA01476.1, glucose dehydrogenase [Priestia megaterium]. Verfügbar unter: https://www.ncbi.nlm.nih.gov/protein/BAA01476.1
Protein [Internet]. Bethesda (MD): National Library of Medicine (US), National Center for Biotechnology Information. Accession No. AHK72173.1, gluconate 5-dehydrogenase Gno [Gluconobacter oxydans DSM 3504]. Verfügbar unter: https://www.ncbi.nlm.nih.gov/protein/AHK72173.1
Protein [Internet]. Bethesda (MD): National Library of Medicine (US), National Center for Biotechnology Information. Accession No. WP_108780707.1, SDR family oxidoreductase [Pontivivens insulae]. Verfügbar unter: https://www.ncbi.nlm.nih.gov/protein/WP_108780707.1
Protein [Internet]. Bethesda (MD): National Library of Medicine (US), National Center for Biotechnology Information. Accession No. CAA46053.1, alcohol dehydrogenase [Thermoanaerobacter brockii]. Verfügbar unter: https://www.ncbi.nlm.nih.gov/protein/CAA46053.1

## Patentansprüche

1. Verfahren zur Herstellung von 5-Keto-D-Gluconat durch Oxidation von D-Glucose, welche in einer wässrigen Lösung vorliegt, **gekennzeichnet durch** die Kombination der Maßnahmen,
dass die D-Glucose durch Behandeln mit einer NAD(P)⁺-abhängigen Glucose-Dehydrogenase und einer NAD(P)⁺-abhängigen Gluconat-5-Dehydrogenase zu 5-Keto-D-Gluconat oxidiert wird, wobei reduzierter Kofaktor NAD(P)H gebildet wird,
dass der reduzierte Kofaktor NAD(P)H enzymatisch mit einer NAD(P)H-Oxidase und/oder einer NAD(P)H-abhängigen Alkoholdehydrogenase zu NAD(P)⁺ oxidiert wird, wobei Sauerstoff und/oder eine Keto-Verbindung als Kosubstrat für die Oxidation des reduzierten Kofaktors eingesetzt wird, und
dass die Glucose-Dehydrogenase, die Gluconat-5-Dehydrogenase und die Keto-Verbindung in der wässrigen Lösung gelöst vorliegen.

2. Verfahren nach Anspruch 1, **dadurch gekennzeichnet, dass** das Verfahren als Eintopf-Verfahren durchgeführt wird.

3. Verfahren nach einem der Ansprüche 1 oder 2, **dadurch gekennzeichnet, dass** eine einzige NAD(P)H-Oxidase und/oder eine einzige NAD(P)H-abhängigen Alkoholdehydrogenase eingesetzt wird.

4. Verfahren nach einem der Ansprüche 1 bis 3, **dadurch gekennzeichnet, dass** eine einzige NAD(P)H-Oxidase eingesetzt wird.

5. Verfahren nach einem der Ansprüche 1 bis 3, **dadurch gekennzeichnet, dass** die Keto-Verbindung Aceton ist.

6. Verfahren nach einem der Ansprüche 1 bis 5, **dadurch gekennzeichnet, dass** das Verfahren in *vitro* durchgeführt wird.

7. Verfahren nach einem der Ansprüche 1 bis 6, **dadurch gekennzeichnet, dass** es in einer einzigen flüssigen Phase durchgeführt wird.
